# FASCICULE DE BREVET EUROPEEN

(11) **EP 1 966 358 B1**
(45) Date de publication et mention de la délivrance du brevet: **21.07.2010**
(21) Numéro de dépôt: 06830026.8
(22) Date de dépôt: 17.11.2006
(51) Int. Cl.: C11B 1/10, C11C 3/00, A23L 1/30, A61K 36/00

(54) **PROCÉDÉ D'OBTENTION D'UNE HUILE D'AVOCAT RAFFINÉE RICHE EN TRIGLYCÉRIDES ET HUILE SUSCEPTIBLE D'ÊTRE OBTENUE PAR UN TEL PROCÉDÉ**
VERFAHREN ZUM ERHALT EINES TRIGLYCERIDREICHEN RAFFINIERTEN AVOCADOÖLS UND NACH EINEM DERARTIGEN VERFAHREN ERHÄLTLICHES ÖL
PROCESS FOR OBTAINING A TRIGLYCERIDE-RICH REFINED AVOCADO OIL, AND OIL WHICH CAN BE OBTAINED BY MEANS OF SUCH A PROCESS

(30) Priorité: 18.11.2005 US 737745 P; 18.11.2005 FR 0511702
(43) Date de publication de la demande: 10.09.2008
(60) Demande divisionnaire: 09153372.9
(73) Titulaire: Laboratoires Expanscience, 92400 Courbevoie (FR)
(72) Inventeur: MSIKA, Philippe, F-78000 Versailles (FR); LEGRAND, Jacques, F-61290 Neuilly Sur Eure (FR)
(74) Mandataire: Warcoin, Jacques
(86) Numéro de dépôt international: PCT/EP2006/068607
(87) Numéro de publication internationale: WO 2007/057439

(56) Documents cités:
- WO-A-2006/004388
- FR-A- 2 653 974
- FR-A- 2 678 632
- FR-A- 2 803 752
- FR-A- 2 806 260
- SOUTHWELL K H, HARRIS R V, SWETMAN A A: "Extraction and refining of oil obtained from dried avocado fruit using a small expeller" TROPICAL SCIENCE, vol. 30, no. 2, 1990, pages 121-131, XP009066801
- ORTIZ MORENO A, DORANTES L, GALINDEZ J, GUZMAN RI: "Effect of different extraction methods on fatty acids, volatile compounds, and physical and chemical properties of avocado (Persea americana Mill.) oil" J. AGRIC. FOOD CHEM., vol. 51, 2003, pages 2216-2221, XP002381544

## Description

La présente invention concerne un procédé d'obtention d'une huile d'avocat raffinée riche en triglycérides. L'invention a également pour objet l'huile d'avocat raffinée riche en triglycérides susceptible d'être obtenue par ce procédé. Avantageusement, l'huile d'avocat raffinée selon l'invention contient une fraction insaponifiable concentrée en stérols. Avantageusement, l'huile d'avocat raffinée selon l'invention est substantiellement exempte d'acétogénines et de lipides furaniques. L'invention concerne également des compositions contenant une telle huile. L'invention a également pour objet de telles compositions pour leur utilisation comme médicament, comme agent dermatologique, comme agent cosmétique, ou comme nutraceutique, à visée humaine ou animale.

L'avocat originaire du Mexique a été ramené en Europe par les conquistadors espagnols. Ce fruit est devenu aujourd'hui un aliment de consommation courante. L'avocat est riche en huile, puisque les lipides représentent entre 10 et 20 % du fruit frais. La grande richesse en corps gras est en effet une des caractéristiques de ce fruit. L'huile, qui par son profil en acides gras est très proche de l'olive, est utilisée depuis de nombreuses années pour ses applications en cosmétique et pharmacie. En outre, grâce à ses propriétés nutritionnelles, son intérêt comme huile alimentaire ou complément alimentaire est apparu depuis ces dernières années, et la production mondiale ne cesse de progresser.

Les huiles brutes ou vierges d'avocat sont généralement extraites par un procédé qualifié de pression à froid, qui consiste en un malaxage et une macération des fruits frais dans l'eau, suivis d'une séparation des trois phases, solide, huileuse et aqueuse dans un décanteur centrifuge. Une ultime phase de polissage par clarification centrifuge permet de produire une huile parfaitement limpide. D'autres procédés, tels que la pression à froid avec addition d'adjuvants solides ou encore l'extraction supercritique, permettent de produire des huiles d'avocat vierges.

Des huiles d'avocat raffinées peuvent être obtenues à partir des huiles vierges ou brutes d'avocat selon les procédés classiques de raffinage, tels que ceux généralement mis en oeuvre pour la production d'huiles de tables conventionnelles, par exemple les huiles de tournesol, de soja, ou de colza. Ce raffinage comprend les opérations suivantes :
- la démucilagination,
- la neutralisation,
- la décoloration,
- la frigélisation,
- la désodorisation.

Un procédé d'extraction d'huile d'avocat par séchage des fruits, extraction par pressage, fractionnement par décantation et raffinage par mise en contact avec des diatomées, traitement alcalin et dégommage est décrit dans la publication SOUTHWELL KH, HARRIS RV, SWETMAN AA : « Extraction and refining of oil obtained from dried avocado fruit using a small expeller » TROPICAL SCIENCE, vol. 30, no. 2,1990, pages 121-131.

La Demanderesse a par ailleurs déjà décrit un procédé pour obtenir une fraction insaponifiable d'huile d'avocat (FR 2 803 752).

Des huiles d'avocat peuvent également être obtenues à partir des avocats séchés. Un traitement thermique des avocats permet de réduire l'humidité résiduelle dans les fruits à une teneur inférieure à 10%, rendant possible l'extraction de l'huile par un solvant organique ou par pression mécanique.

Cependant, de tels procédés d'extraction incluant un chauffage de l'avocat et une extraction par solvant ou par pression mécanique, entraînent une modification de la composition de l'huile extraite. Lors du chauffage de l'avocat, des réactions chimiques libèrent des composés qui sont partiellement transformés et extraits avec l'huile. Ces composés sont instables et ne sont pas totalement éliminés par les procédés de raffinage classiques. L'instabilité de ces composés rend difficile leur élimination lors du raffinage sans risque de transformation en molécules non maîtrisées.

Par ailleurs, ces procédés ne permettent pas de préparer des huiles d'avocats raffinées garantissant une parfaite innocuité pour une utilisation alimentaire ou nutraceutique. En effet, des études ont montré que les huiles brutes obtenues à partir d'avocats séchés incluant notamment le noyau contenaient des composés pouvant présenter une toxicité. Werman, M.J. ; Mokady, S. ; Néeman, I ; Auslaender, L. ; Zeidler, A. Food and chem. Toxicol., 1989, 27, 279. Ainsi, on retrouve de nombreux constituants spécifiques de l'avocat, tels que les acétogénines qui sont des composés toxiques détectés dans les fruits et les feuilles d'avocat, dans les huiles d'avocat extraites et raffinées par les procédés de l'art antérieur.

Ainsi, lorsque l'on met en oeuvre les procédés conventionnels de raffinage incluant démucilagination, neutralisation, décoloration, frigélisation et désodorisation, les différents traitements ne sont pas suffisamment efficaces pour garantir l'élimination totale des composés instables ou leur non dégradation en dérivés secondaires.

Ainsi, la faible stabilité de certains constituants de l'avocat et les risques liés à leur toxicité potentielle nécessitent la maîtrise du devenir de ces constituants lors des différentes étapes du procédé pour produire une huile de qualité alimentaire. Par conséquent, il existait un besoin de mettre au point un nouveau procédé visant à obtenir des huiles d'avocat raffinées ne présentant pas les inconvénients de l'art antérieur et permettant de commercialiser des huiles d'avocats séchés en toute sécurité.

La présente invention vient combler ce besoin. La Demanderesse a ainsi découvert un nouveau procédé d'obtention d'une huile d'avocat raffinée, très simple à mettre en oeuvre, comprenant la combinaison de différentes opérations qui permettent de maîtriser et contrôler précisément l'évolution et la transformation des différents constituants spécifiques de l'avocat. Le procédé selon la présente invention permet de se débarrasser substantiellement des impuretés spécifiques de l'avocat et de garantir son innocuité. Ainsi, le séchage des fruits avant extraction de l'huile, et la réalisation d'une étape de fractionnement de l'huile, avantageusement par distillation moléculaire, permet de se débarrasser substantiellement des produits potentiellement toxiques de l'avocat, tels que les acétogénines encore appelés « persin », et des produits de dégradation responsables de l'amertume de structure furanique, en particulier les lipides furaniques.

Avantageusement selon la présente invention, l'étape de distillation moléculaire judicieusement intercalée entre le séchage des fruits et le raffinage de l'huile permet l'élimination des composés non désirés et instables. Le procédé selon l'invention, incluant notamment une étape de distillation moléculaire en amont du raffinage, conduit à la préparation d'une fraction enrichie en triglycérides et débarrassée substantiellement de tout composé présentant un risque de toxicité ou pouvant conduire à la formation de dérivés responsables de l'amertume de l'huile raffinée.

Par ailleurs, le procédé selon la présente invention permet l'obtention, avec un rendement élevé, d'une huile d'avocat raffinée riche en triglycérides, et contenant une fraction insaponifiable qui est enrichie en stérols. L'huile obtenue par un tel procédé, ou susceptible d'être obtenue par un tel procédé, peut ainsi avantageusement être incorporée au sein de compositions cosmétiques, dermatologiques ou pharmaceutiques, ou encore dans des compositions alimentaires, des compléments alimentaires, ou des nutraceutiques, à visée humaine ou animale. L'huile selon la présente invention présente également l'avantage d'être parfaitement limpide et claire, et peut ainsi être très facilement incorporée dans divers types de compositions.

En outre, l'huile d'avocat raffinée obtenue selon le procédé de l'invention, ou susceptible d'être obtenue par un tel procédé, est riche en acide gras monoinsaturés qui sont particulièrement intéressants pour leur action reconnue sur l'abaissement du taux du LDL-cholestérol dans le sang. L'huile selon l'invention est donc particulièrement appropriée pour réduire les problèmes de santé cardiovasculaire. L'huile d'avocat raffinée selon l'invention, riche en triglycérides, lui garantit une stabilité renforcée de par sa teneur faible en mono et diglycérides.

La présente invention a ainsi pour objet un procédé d'obtention d'une huile d'avocat raffinée riche en triglycérides, caractérisé en ce qu'il comprend les étapes successives suivantes :
(1) la déshydratation contrôlée des avocats frais ou ayant subi des transformations préalables, avantageusement réalisée à une température comprise entre -50°C et 120°C, en particulier comprise entre -50°C et 90°C,
(2) l'extraction de l'huile des fruits déshydratés,
(3) le fractionnement de l'huile en sa fraction riche en triglycérides, puis
(4) le raffinage de la fraction de l'huile riche en triglycérides.

L'huile d'avocat raffinée contient avantageusement une fraction insaponifiable riche en stérols.

Par le terme d'" huile d'avocat raffinée riche en triglycérides ", on entend au sens de la présente invention une huile d'avocat raffinée contenant au moins 80% en poids de triglycérides, avantageusement au moins 90% en poids de triglycérides, encore plus avantageusement de 95% à 99% en poids de triglycérides, par rapport au poids total de l'huile d'avocat raffinée.

De manière particulièrement avantageuse selon la présente invention, l'huile d'avocat raffinée ne contient pas plus de 1 % en poids de monoglycérides, de préférence pas plus de 0,1% en poids de monoglycérides, par rapport au poids total de l'huile d'avocat raffinée. Avantageusement, la teneur en diglycérides présents dans l'huile de la présente invention est comprise entre 1 et 5 % en poids, et préférentiellement entre 1 ,5 et 3 % en poids.

Par « avocat ayant subi des transformations préalables », on entend les co-produits issus des procédés d'extraction des huiles d'avocat frais, notamment ceux issus des procédés dits de centrifugation. Ainsi, on peut notamment citer, à titre d'« avocat ayant subi des transformations préalables », i) les laits d'avocat obtenus par pressage des pulpes, ou encore ii) les produits de débourbage des pulpes partiellement déshuilées par centrifugation, sous-produits généralement présents en sortie des passoires centrifuges, ou encore les culots de centrifugeuses produits au cours de la séparation.

D'autres sources d'avocat, que l'on fait entrer dans le terme « avocat ayant subi des transformations préalables » peuvent encore être citées : Ainsi, les tourteaux d'avocat, co-produits lors de la pression à froid des fruits (frais ou séchés) ou de l'extraction liquide-solide de l'huile d'avocat de fruits frais ou séchés, à l'aide d'un solvant organique, ou les sous produits de préparation alimentaire à base d'avocat du type guacamole, peuvent aussi constituer en l'état, une matière première alternative utilisable dans le cadre de la présente invention.

Avantageusement selon la présente invention, préalablement à l'étape de déshydratation (1), les fruits d'avocat fraîchement récoltés subissent une étape de tri pour éliminer les fruits trop matures, abîmés ou présentant des moisissures. Les avocats engagés sont ensuite découpés et sont de préférence répartis en couches les plus régulières et fines possibles, afin de faciliter un séchage et/ou une déshydratation rapide et homogène.

On entend plus généralement par déshydratation, effectuée à l'étape (1) du procédé, l'ensemble des techniques connues de l'homme de métier qui permettent d'extraire l'eau d'un composé. Parmi ces techniques, on peut citer le séchage sous courant d'air chaud ou sous atmosphère contrôlée (ex. azote), le séchage à pression atmosphérique ou sous vide, le séchage en couche épaisse ou couche mince, ainsi que le séchage par micro-ondes, le séchage par pulvérisation, la lyophilisation ou encore la déshydratation osmotique en solution (ex. osmose directe) ou en phase solide (ex. séchage en sacs osmotiques).

Dans un mode de réalisation particulier de la présente invention, la déshydratation (1) consiste en un séchage des fruits tranchés, de préférence réalisé dans un séchoir à air chaud à une température comprise entre 70 et 90°C, en particulier entre 75 et 80°C. La durée du séchage est avantageusement comprise entre 8 et 36 heures jusqu'à l'obtention d'un taux résiduel d'humidité dans le fruit de préférence inférieur ou égal à 5 % en sortie de séchoir.

Dans le cadre du présent procédé, pour des raisons de facilité de mise en oeuvre industrielle et pour des raisons de coût, le séchage en séchoirs ventilés, en couche mince et sous courant d'air chaud, à une température comprise entre 70 et 80°C pendant 8 à 36 heures, est préféré.

Lorsque la déshydratation (1) est réalisée par séchage, à une température supérieure à la température ambiante, ce traitement thermique de l'avocat, mené dans des conditions bien définies, permet de favoriser la transformation moléculaire de composés spécifiques de l'avocat et d'optimiser le rendement de transformation tout en évitant la synthèse de produits de dégradation thermique ou d'oxydation.

L'étape (2) d'extraction peut être mise en oeuvre par tout moyen connu de l'homme de métier, de préférence par une simple pression à froid ou à l'aide d'un solvant à basse température. La pression mécanique des fruits déshydratés permet de garantir la qualité de l'huile et de ses constituants insaponifiables, et d'obtenir un taux de récupération élevé de l'huile sans emploi d'un produit organique tel qu'un solvant.

Dans un mode de réalisation particulier de la présente invention, les lamelles d'avocats déshydratés, de préférence par séchage, sont broyées à l'aide d'un broyeur mécanique. La poudre obtenue est alors alimentée par l'intermédiaire d'un pré-cuiseur dans une presse mécanique à vis continue. L'huile produite par pression mécanique est décantée, puis filtrée sur filtre presse.

L'étape (3) de fractionnement peut être mise en oeuvre par tout moyen connu de l'homme de métier, et consiste avantageusement en une cristallisation à froid, une distillation sous vide, ou une distillation moléculaire. Cette étape de fractionnement permet de séparer d'une part une fraction riche en insaponifiables, et d'autre part une fraction lourde riche en triglycérides. Typiquement, la fraction riche en insaponifiables représente 8 à 15 % en poids de l'huile brute et la fraction riche en triglycérides représente 85 à 92 % en poids de l'huile brute.

L'insaponifiable est la fraction d'un corps gras qui, après action prolongée d'une base alcaline, reste insoluble dans l'eau et peut être extraite par un solvant organique. Cinq grands groupes de substances sont présents dans la plupart des insaponifiables d'huiles végétales : hydrocarbures saturés ou insaturés, alcools aliphatiques ou terpéniques, stérols, tocophérols, les pigments caroténoïdes et xanthophylles.

La fraction riche en insaponifiables contient en général 30 à 80% en poids d'insaponifiables. La fraction riche en triglycérides contient en général au moins 80% en poids, avantageusement au moins 90% en poids, en particulier de 95 à 99 % en poids de triglycérides. La fraction riche en triglycérides contient elle-même également des insaponifiables, en général de 0,5 à 1,5 % en poids, par rapport au poids total de la fraction riche en triglycérides. Ces insaponifiables sont concentrés en stérols.

L'étape (3) de fractionnement, avantageusement réalisée par distillation moléculaire, permet de se débarrasser substantiellement des composés spécifiques de l'avocat, tels que les acétogénines potentiellement toxiques, et de leurs composés de transformation ou de dégradation, notamment les lipides furaniques responsables de l'amertume.

Cette étape (3) permet également d'isoler sans dégradation thermique la fraction concentrée en insaponifiables d'une part, et la fraction concentrée en triglycérides d'autre part. Par ailleurs, elle permet d'abaisser fortement la teneur en acides gras de la fraction riche en triglycérides, facilitant l'étape ultérieure de raffinage telle que la neutralisation, et limitant les pertes de produits.

Un avantage supplémentaire du fractionnement (3), de préférence réalisé par distillation moléculaire, consiste en son pouvoir de fractionnement des constituants des composés insaponifiables. Lors de l'étape de distillation, les stérols, présents sous forme d'esters d'acides gras, contrairement aux stérols non estérifiés et à tous les autres constituants des insaponifiables, ne possèdent pas un point d'ébullition suffisamment bas pour être distillés. Les composés stéroliques estérifiés se retrouvent donc concentrés dans la partie des insaponifiables contenus dans la fraction lourde riche en triglycérides de l'huile d'avocat. Les stérols sous forme d'esters de stérol forment ainsi les composés majeurs des insaponifiables suite à l'étape de fractionnement de l'huile.

Selon une caractéristique particulière de la présente invention, le fractionnement (3) est réalisé par distillation moléculaire, permettant la séparation presque intégrale des composés de l'insaponifiable, la désacidification de la fraction triglycéridique, et l'enrichissement en esters de stérols de la partie insaponifiable de la fraction triglycéridique.

Avantageusement selon la présente invention, l'huile brute issue de l'étape d'extraction (2) est pompée vers un dégazeur continu, préalablement à l'étape de fractionnement (3). Le dégazeur continu est un appareil à film tombant qui permet, avant distillation, d'éliminer les éventuelles traces d'eau et de gaz dissous.

Selon une caractéristique particulière de la présente invention, le fractionnement (3) est une distillation moléculaire, qui peut être réalisée à une température comprise entre 180 et 260°C, avantageusement entre 200 et 250°C, encore plus avantageusement entre 220 et 230°C, en maintenant une pression comprise entre 0.133 et 13.3 Pa, avantageusement entre 0.133 et 1.33 Pa (10⁻³ et 10⁻¹ mmHg, avantageusement entre 10⁻³ et 10⁻² mmHg).

Cette étape de distillation moléculaire est de préférence réalisée en utilisant un dispositif choisi parmi les distillateurs moléculaires de type centrifuge et les dispositifs moléculaires de type à film raclé.

Les distillateurs moléculaires de type centrifuge sont connus de l'homme du métier. Par exemple, la demande EP 493 144 décrit un distillateur moléculaire de ce type. D'une manière générale, le produit à distiller est étalé en couche mince sur la surface chauffée (surface chaude) d'un rotor conique tournant à grande vitesse. L'enceinte de distillation est placée sous vide. Dans ces conditions, il y a évaporation et non pas ébullition, depuis la surface chaude, des constituants de l'huile tels que les insaponifiables, l'avantage étant que l'huile et ses constituants, notamment les insaponifiables (ces produits étant réputés fragiles), ne sont pas dégradés au cours de l'évaporation.

Les distillateurs moléculaires de type à film raclé, également connus de l'homme du métier, comprennent une chambre de distillation dotée d'un racleur tournant, permettant l'étalement en continu sur la surface d'évaporation (surface chaude) des produits à distiller. Les vapeurs de produit sont condensées par le biais d'un doigt réfrigéré, placé au centre de la chambre de distillation. Les systèmes périphériques d'alimentation et de vide sont très proches de ceux d'un distillateur centrifuge (pompes d'alimentation, pompes à vide à palette et à diffusion d'huile, etc.). La récupération des résidus et des distillats dans des ballons en verre se fait par écoulement gravitationnel.

A l'issue de l'étape de fractionnement (3), la fraction distillée riche en insaponifiables représente en général 5 à 15 % en poids de l'huile de départ, et la fraction distillée riche en triglycérides représente en général 85 à 95 % en poids de l'huile de départ.

La fraction riche en triglycérides est ensuite purifiée par raffinage.

L'étape (4) de raffinage comprend avantageusement au moins une des opérations suivantes : traitement acide, neutralisation chimique, décoloration, frigélisation, et désodorisation. Cette étape de raffinage permet de purifier la fraction glycéridique lourde afin d'obtenir une huile d'avocat raffinée contenant une fraction insaponifiable riche en stérols, qui va pouvoir être utilisée pour des applications cosmétiques, pharmaceutiques, alimentaires ou nutraceutiques, à visée humaine ou animale.

De façon particulièrement avantageuse, l'étape (4) de raffinage comprend l'ensemble des opérations successives suivantes : traitement acide, neutralisation chimique, décoloration, frigélisation, et désodorisation.

Le traitement acide est de préférence un conditionnement acide, permettant de réaliser une démucilagination et d'éliminer les phospholipides. Le traitement acide est typiquement réalisé à l'aide d'un acide faible, tel que l'acide phosphorique ou l'acide citrique, ou à l'aide d'un acide fort, tel que l'acide sulfurique ou l'acide chlorhydrique. Généralement, le traitement acide est réalisé sous agitation, à une température comprise entre 30 et 70°C, typiquement aux alentours de 50 à 60°C.

D'autres procédés peuvent être préférés, tels que la microfiltration qui est un procédé membranaire basse pression, utilisé pour la filtration des colloïdes, ou des procédés faisant appel à la biotechnologie tels que la démucilagination par voie enzymatique.

La neutralisation chimique, qui suit typiquement le traitement acide, permet d'éliminer les acides gras libres, de débarrasser l'huile des phospolipides qui ont subi l'opération de conditionnement, d'éliminer les traces métalliques, et de faciliter la décoloration en détruisant un certain nombre de pigments et de composés colorés d'origine oxydative. La neutralisation chimique est typiquement réalisée à l'aide d'un agent basique tel que la soude, le carbonate de potassium ou une amine tertiaire (DMHA). Les acides gras sont séparés, par centrifugation ou filtration, sous forme de savons qui contiennent également les mucilages et diverses impuretés éliminés lors de ce traitement.

De façon avantageuse selon la présente invention, on réalise un lavage, de préférence à l'eau, suite à l'opération de neutralisation chimique. De préférence, on réalise ensuite une étape de séchage du milieu réactionnel, généralement sous vide à haute température, par exemple à 90°C.

La décoloration, qui suit typiquement la neutralisation chimique, permet en général d'éliminer les pigments colorés que la neutralisation n'a que très partiellement détruits. La décoloration est typiquement mise en oeuvre à l'aide de terres décolorantes et/ou de charbon, avantageusement jusqu'à l'obtention d'une couleur claire ou très claire de l'huile d'avocat.

Les terres décolorantes utilisées sont typiquement des argiles naturelles du type montmorillonite, constituées principalement d'aluminosilicates de calcium et magnésium, et activées par traitement acide. Les charbons actifs utilisés peuvent être obtenus à partir de tourbe, bois, lignite, charbon ou de coques de noix de coco. Ces produits sont typiquement activés à haute température, soit à la vapeur, soit par un procédé chimique.

L'étape de frigélisation, qui suit typiquement la décoloration, peut être réalisée à une température comprise entre 5 et 18 °C, avantageusement pendant 1 à 15 jours. Typiquement, l'huile d'avocat raffinée est refroidie progressivement, de préférence sous agitation lente, jusqu'à une température de l'ordre de 10 à 12 °C. Généralement, l'huile est alors maintenue à cette température pendant 48 h, puis est filtrée sous pression. La frigélisation ou wintérisation permet de précipiter les triglycérides riches en acides gras saturés. Cette étape est particulièrement avantageuse puisqu'elle permet l'obtention d'une huile d'avocat raffinée limpide et claire, qui ne se trouble pas à basse température, garantissant ainsi un aspect homogène, quelles que soient ses conditions de conservation.

La désodorisation, qui suit typiquement la frigélisation, permet en général d'éliminer et d'extraire les composés volatils et les molécules malodorantes. La désodorisation est typiquement réalisée à une température comprise entre 150 et 210 °C, avantageusement sous vide, généralement sous courant de vapeur saturée ou d'azote. La désodorisation peut par exemple être réalisée sous une pression comprise entre 2 et 20 mmHg. En particulier, la désodorisation peut être effectuée à une température de l'ordre de 180 à 200°C, sous une pression de l'ordre de 4 à 6 mmHg. Le pourcentage de vapeur injectée représente généralement 0,5 à 4 % de l'huile traitée. Le temps d'injection peut être compris entre 2 et 6 heures. Après séchage et refroidissement, l'huile d'avocat raffinée est alors avantageusement conditionnée sous gaz inerte, à l'abri de la lumière et de l'humidité.

La présente invention a également pour objet l'huile d'avocat raffinée riche en triglycérides, susceptible d'être obtenue par le procédé de la présente invention. Avantageusement, cette huile contient une fraction insaponifiable riche en stérols.

Selon une caractéristique particulière de la présente invention, une telle fraction insaponifiable contient au moins 40% en poids de stérols, avantageusement entre 45 et 70% en poids de stérols, par rapport au poids total des insaponifiables.

De manière particulièrement avantageuse, l'huile selon la présente invention est substantiellement exempte d'acétogénines (persin) ou de lipides furaniques.

Par le terme de « substantiellement exempte d'acétogénines ou de lipides furaniques », on entend au sens de la présente invention une huile ne contenant pas une teneur quantifiable en acétogénines ou en lipides furaniques.

Avantageusement, cette fraction ne contient pas de teneur quantifiable en persin ou en lipides furaniques. Le fait d'obtenir une huile substantiellement exempte d'acétogénines est particulièrement avantageux, puisque les acétogénines sont des composés potentiellement toxiques et thermiquement instables. En effet, sous l'effet de la chaleur, ces composés conduisent à la formation de lipides furaniques responsables de l'amertume de l'huile.

L'huile d'avocat raffinée riche en triglycérides selon la présente invention contient au plus 100 ppm d'acétogines et au plus 500 ppm de lipides furaniques, par rapport au poids total de l'huile.

L'avocat comprend de manière connue des lipides particuliers de type acétogénines aliphatiques, dont le principal composant est un acétogénine linoléique répondant à la formule : 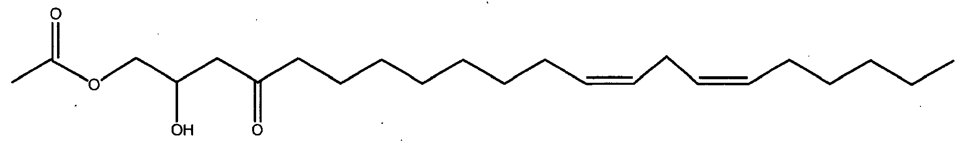 Ces composés sont aussi communément appelés du nom générique de persin. Ainsi, par «acétogénines aliphatiques», on entend selon l'invention les composants répondant à la formule : 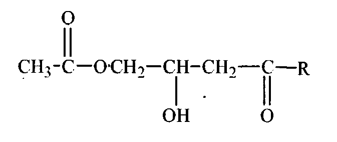 dans laquelle R est une chaîne linéaire hydrocarbonée en C₁₁-C₁₉, de préférence C₁₃-C₁₇, saturée ou comprenant une ou plusieurs insaturation(s) éthylénique(s) ou acétylénique(s).

« Par lipides furaniques d'avocat », on entend selon l'invention les composants répondant à la formule : 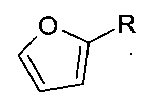 dans laquelle R est une chaîne linéaire hydrocarbonée en C₁₁-C₁₉, de préférence C₁₃-C₁₇, saturée ou comprenant une ou plusieurs insaturation(s) éthylénique(s) ou acétylénique(s).

Avantageusement, la fraction insaponifiable de l'huile selon l'invention est riche en stérols, et l'huile peut ainsi être utilisée pour abaisser le taux de cholestérol sanguin.

Cette huile possède également une forte teneur en acides gras monoinsaturés. L'acide oléique représente près des 2/3 des acides gras de l'avocat. Ainsi, l'huile selon l'invention est avantageusement utilisée dans la prévention des maladies cardio-vasculaires.

En outre, la teneur élevée en triglycérides de l'huile et sa faible teneur en glycérides partiels (mono et di) mettent en évidence une grande pureté de l'huile et garantissent un degré d'hydrolyse faible et une bonne stabilité.

La présente invention a également pour objet une composition contenant une telle huile d'avocat raffinée riche en triglycérides. Avantageusement, cette huile est présente à une concentration comprise entre 5 et 95 % en poids, avantageusement entre 10 et 40 % en poids, par rapport au poids total de la composition.

De manière particulièrement avantageuse, les compositions selon la présente invention comprennent en outre au moins un composé choisi parmi les huiles végétales, les insaponifiables d'huile végétale, les lipides furaniques d'huile végétale, et leurs mélanges.

Parmi les huiles végétales pouvant être utilisées, on peut citer en particulier l'huile de tournesol, de palme, de palmiste, de noix de coco, de pépins de raisins, de moutarde noire, d'oeillette, de beurre de karité, d'amande douce, de soja, d'avocat, d'arachide, de coton, de sésame, d'olive, de maïs, de cacao, de ricin, de Ben, de lin, de colza, de rocouyer, de germe de blé, de carthame, de lupin, de noix, de noisette et de navette. Les huiles végétales, dont l'insaponifiable est riche en tocophérols et/ou en phytostérols, sont particulièrement préférées. C'est le cas par exemple du soja, du maïs, du colza, ou du tournesol.

Avantageusement, les compositions contiennent une huile d'avocat raffinée riche en triglycérides selon la présente invention en association avec de l'huile d'avocat issue de fruits frais.

Les compositions de la présente invention peuvent également comprendre des huiles d'origine animale ou des composés purifiés riches en oméga-3 (acide eicosapentanoïque, acide docohexanoïque, acide alpha-linolénique, ...), tels que les huiles de poisson ou l'huile de graines de lin.

Les compositions de la présente invention peuvent également comprendre des oméga-6 tels que l'acide gamma-linolénique. A ce titre, les compositions de la présente invention peuvent contenir notamment de l'huile de bourrache, de l'huile de cassis, ou d'onagre (par exemple de 200 à 500 mg/j).

Les compositions de la présente invention peuvent également comprendre au moins un composé choisi parmi : pema canaliculus, yucca, boswelia , les acides aminés, les peptides synthétiques et végétaux, les protéines animales et végétales, la bromélaine, et les levures.

Les compositions de la présente invention peuvent également comprendre des agonistes PPARs naturels ou de synthèse. Ces agonistes PPARs naturels ou de synthèse sont avantageusement choisis parmi : les ligands naturels, en particulier les leucotriènes, le 8S-HETE, l'acide phytanique, les acides gras insaturés et saturés, la prostaglandine, (15d-PGJ2), 9-HODE, 13-HODE ; les ligands synthétiques, en particulier tous les composés de la famille des fibrates (par exemple le fénofibrate), les tibrates, les anti-inflammatoires notamment l'indométhacine, l'acide flufénamique, l'ibuprofène, et le fénoprofène, et les glitazones dont les thiazolidinediones (troglitazone, rosiglitazone, pioglitazone, et darglitazone), et leurs mélanges.

Les insaponifiables d'huile végétale pouvant être incorporés dans les compositions selon l'invention sont de préférence choisis dans le groupe constitué par l'insaponifiable d'huile d'avocat, l'insaponifiable d'huile de soja, et les mélanges de ces derniers.

A titre d'exemple selon la présente invention, on peut mentionner une composition contenant environ 17% en poids d'huile d'avocat raffinée riche en triglycérides selon la présente invention, environ 33% en poids d'insaponifiables d'huile de soja, et environ 50% en poids d'une huile de soja raffinée disponible commercialement. Cette composition peut également contenir des excipients. Cette composition est avantageusement sous forme de gélule de 300 mg à 5 g.

De manière particulièrement avantageuse, la composition selon la présente invention contient l'huile d'avocat raffinée riche en triglycérides selon la présente invention, ainsi que des insaponifiables d'huile de soja. Par exemple, la composition contient environ 33% en poids d'huile d'avocat raffinée riche en triglycérides selon la présente invention, et environ 67% en poids d'insaponifiables d'huile de soja, éventuellement avec des excipients. Cette composition est avantageusement sous forme de gélule de 300 mg à 5g.

Les compositions selon l'invention sont avantageusement utilisées sous forme de comprimé appétent pour animaux, ou sous forme de gel alimentaire pour chevaux.

Les constituants des insaponifiables d'huiles végétales sous forme concentrée ou purifiée peuvent être incorporés dans les compositions selon l'invention, et sont choisis de préférence dans le groupe constitué par les phytostérols et les esters de phytostérols, les tocophérols et les esters de tocophérols, les tocotriénols et les esters de tocotriénols, le squalène, la fraction I de l'avocat (alcools 1,2,4-trihydroxyaliphatiques de l'avocat), les alcools triterpéniques et les esters des alcools triterpéniques, les stanols et les esters de stanols, les alcools aliphatiques et les esters d'alcools aliphatiques, et leurs mélanges.

Les compositions de la présente invention peuvent également comprendre des composés tels que le produit Avosoy du Docteur Théo, contenant notamment un mélange d'acides gras, des insaponifiables (squalène, tocophérols, stérols), et des stérols.

Les compositions de la présente invention peuvent également comprendre des composés tels que le produit Avoflex de Cyvex, contenant notamment un mélange d'acides gras, des insaponifiables (squalène, tocophérols, stérols) et des stérols.

Les compositions de la présente invention peuvent également comprendre des composés tels que les produits Vegeflex et Prevention de Windmill, contenant notamment un mélange d'acides gras, des insaponifiables, des tocophérols, et des stérols, avantageusement en association avec de la glucosamine.

Les compositions de la présente invention peuvent également comprendre des composés tels que le produit Avosol de A/S Anjo, contenant notamment des insaponifiables.

Dans un mode de réalisation particulier de la présente invention, les compositions comprennent en outre au moins un composé choisi parmi les sucres aminés tels que la glucosamine ; les sels de glucosamine, tels que le chlorhydrate de glucosamine (par exemple de 1500 à 2000 mg/j), le sulfate de glucosamine, le phosphate de glucosamine et le N-acétylglucosamine ; les glycosaminoglycanes (GAG) tels que la chondroïtine sulfate (par exemple de 800 à 1200 mg/j) ; les analogues de glycosaminoglycanes tels que les glycosaminoglycanes polysulfatés, ou les précurseurs de glycosaminoglycanes tels que l'acide hyaluronique, l'acide glucuronique, l'acide iduronique, le keratane sulfate, le heparane sulfate, ou le dermatine sulfate ; la pentosane ou ses dérivés, en particulier la pentosane sulfate, la pentosane polysulfate (PPS) et les polysaccharides pentosane polysulfates ; la S-adénosylméthionine (SAMe) ; l'adénosine ; la superoxyde dismutase (SOD) ; la L-ergothionine ; les collagènes de type II hydrolysés ou non ; les hydrolysats de collagène tels que la gélatine ; la diacérine ; l'acide arachadonique ; la tétracycline ; les composés analogues de la tétracycline ; la doxycycline ; l'hydroxyproline ; et leurs mélanges.

Avantageusement, les compositions de la présente invention comprennent en association plusieurs des composés mentionnés ci-dessus. La glucosamine et la chondroïtine sulfate, seuls ou en association, sont des composés particulièrement préférés.

Dans un mode de réalisation particulier de la présente invention, les compositions comprennent en outre au moins un composé choisi parmi les extraits de céleri, de moule verte, de concombre de mer, ou de cartilage ovin, bovin, caprin ou marin (requin), les extraits de kératine classique ou de keratec (cynatine FLX), la Sulfate AdenosineMethionine, l'acide hyaluronique de tous les poids moléculaires, les vitamines A (5000UI), vitamines C (jusqu'à 1g), vitamines E (100à400UI), le sélénium de 55 à 200 mcg), les flavonoïdes et les composés alimentaires en contenant tels que : rutine, extrait de phellodendron amurensi, curcumin, ail, luteine, zeaxanthine, lycopène, hesperidine, gingko, OPC (le tout jusqu'à 1g/j), EPA d'algues de plancton, de krill, de poisson (jusqu'à 2g /j), DHA, LA (acide linoléique), ou ALA.

Les compositions de la présente invention peuvent également comprendre des composés tels que l'hormone de croissance (somatotrophine), les somatomédines, les facteurs de croissance insulinoïdes, ainsi que des analgésiques du type acétaminophène ou tramadol, ou encore des anti-inflammatoires non stéroïdiens ou des anti-COX2.

Les compositions de la présente invention peuvent également comprendre des oligo-éléments tels que le bore, le cuivre, le magnésium, le manganèse, le sélénium (par exemple de 55 à 200 mg), la silice, le zinc, et leurs dérivés.

Les compositions de la présente invention peuvent également comprendre des vitamines (A, C, D, E, K, B3, B5, B6, B12).

Les compositions de la présente invention peuvent également comprendre des composés capables de limiter la dégradation osseuse et/ou de favoriser la reminéralisation de l'os, tels que les biphosphonates, le calcium, les bêta-bloquants, les phytoestrogènes, le raloxifène, ou encore le strontium.

Les compositions de la présente invention peuvent également comprendre des extraits de plantes, notamment de broméaline, harpagophytum, gingembre, ginkgo biloba, centellia asiatica, kava-kava, valériane, wild yam, ou de céléri.

Les compositions peuvent également comprendre des anti-oxydants tels que le glutathion; la N-acétyl-cystéine, le MSM (méthyl sulfonide méthane), les extraits de thé, notamment de thé vert, le sulphoraphane issu du brocoli, ou des extraits aqueux de grenade.

Les compositions de la présente invention peuvent également comprendre des hormones, telles que la mélatonine ou la DHEA.

Avantageusement, les compositions de la présente invention comprennent également des inhibiteurs de métalloprotéases matricielles (MMPs), tels que les peptides de lupin.

Par « composés inhibiteurs des métalloprotéases matricielles (MMPs) », on entend selon l'invention tout composé connu de l'homme du métier pour sa capacité d'inhiber l'activité de dégradation de la matrice extracellulaire par les MMPs. Les MMPs constituent une famille d'enzymes (actuellement plus d'une vingtaine ont été identifiées et caractérisées), zinc-dépendantes, de structure très conservée, qui possèdent la capacité de dégrader les composants de la matrice extracellulaire. Elles sont classées selon la nature de leur substrat en collagénases, gélatinases et stromélysine. Le groupe des MMPs est ainsi constitué de quatre sous-classes : (1) les collagénases, (2) les gélatinases, (3) les stromelysines, et (4) les MMPs de type membranaires (MT-MMPs). L'activité des MMPs peut être modulée par des inhibiteurs de protéinase naturellement présents, tels que les inhibiteurs tissulaires de métalloprotéinases (TIMPs ; notamment les TIMP-1 et TIMP-2). En particulier, on utilise un composé actif pour inhiber au moins une MMP choisie dans le groupe constitué par les MMP-1, MMP-2, MMP-3 MMP-9, MMP-7, MMP-13 et MMP-18. Comme « composé inhibiteur des MMPs », on entend en particulier les inhibiteurs tissulaires de métalloprotéinases (TIMPs), l'alpha-2-macroglobuline, les inhibiteurs de l'activateur du plasminogène, les chélateurs de zinc, la bryostatine-1, les antibiotiques (doxycyclines, minocyclines, etc.), les peptides synthétiques ou naturels ayant une structure similaire aux substrats des MMPs (batimastat, marimastat, etc.), les rétinoïdes (en particulier les rétinoïdes non aromatiques tels le rétinaldéhyde, la trétinoïne, et l'acide rétinoïque 9-cis, la vitamine A, les rétinoïdes monoaromatiques tels l'étrétinate, l'all-trans acitretine et le motrerinide, et les rétinoïdes polyaromatiques tels que l'adapalène, le tazarotène, le tamibarotène et l'arotinoïde methyl sulfone), les antioxydants (les piègeurs d'oxygène singulet, etc.), les anti-cancéreux (ou « anti-métastatiques »), les hydrolysats de malt tels que Colalift commercialisés par la société Coletica, les extraits d'algues marines tels que Kelpadélie commercialisés par la société Secma, les extraits de cartilage de requin tels que le complexe MDI commercialisés par la société Atrium, les peptides de riz comme par exemple Colhibin commercialisé par la société Pentapharm, les extraits peptidiques de lupin. Plus particulièrement, le composé inhibiteur des MMPs selon la présente invention est choisi dans le groupe constitué par les extraits peptidiques de lupin ou « peptides de lupin », tels que ceux décrits dans la demande de brevet FR-99 04 875 déposée le 19 avril 1999 au nom de la société Laboratoires Expanscience. On peut notamment citer l'extrait peptidique décrit dans la demande FR 99 04875 sous la dénomination extrait B (LU105). Selon un autre mode préféré de réalisation, les compositions selon l'invention contiennent l'inhibiteur des MMPs choisi dans le groupe constitué par les rétinoïdes.

La présente invention a également pour objet lesdites compositions ou l'huile d'avocat raffinée de l'invention pour leur utilisation comme médicament, comme agent dermatologique, comme agent cosmétique, ou comme nutraceutique, à visée humaine ou animale. Lesdites compositions ou l'huile d'avocat raffinée selon l'invention sont avantageusement utilisées dans des applications vétérinaires.

L'huile d'avocat raffinée et les compositions selon la présente invention peuvent être administrées à l'être humain ou l'animal, avantageusement par voie topique ou orale.

Les compositions selon la présente invention peuvent en particulier se présenter sous toute forme galénique appropriée, et notamment sous forme de lotion aqueuse ou hydroalcoolique, monophasique ou polyphasique, de gel monophasique ou polyphasique, d'émulsion, de crème, de dispersion vésiculaire, de mousse ou de spray.

De manière particulièrement avantageuse, l'huile d'avocat raffinée ou les compositions selon la présente invention peut (peuvent) également être utilisée(s) dans la prévention ou le traitement des maladies cardiovasculaires, des pathologies articulaires telles que l'arthrose, des rhumatismes, des maladies parodontales, telles que la gingivite ou la parodontite, des inflammations, ou encore dans la prévention ou le traitement de la dégradation de la matrice extracellulaire, dans la prévention ou le traitement du vieillissement de la peau, ou encore pour le bien être des gencives, des cartilages et de la peau.

L'huile d'avocat raffinée selon la présente invention, de par son profil en acides gras, est très proche de l'huile d'olive. De par son arôme, sa résistance à la chaleur et ses qualités nutritionnelles, l'huile d'avocat peut être utilisée pour des usages culinaires à froid (salades, vinaigrettes, mayonnaises...) ou à chaud (huile de cuisson, friture...).

L'utilisation en association avec des arômes ou des extraits végétaux permet de mettre en valeur les qualités nutritionnelles de cette huile et son originalité pour les préparations culinaires.

L'huile d'avocat raffinée selon la présente invention possède une forte teneur en acides gras monoinsaturés. Il est maintenant reconnu qu'une diète riche en acides gras monoinsaturés réduit les taux de cholestérol total et de mauvais cholestérol (LDL), et augmente celui de bon cholestérol (HDL). Depuis les années 50, plus de 600 études scientifiques ont permis de démontrer qu'un régime riche en phytostérols aide à contrôler les taux sanguins de cholestérol total et de LDL-cholestérol chez l'animal et chez l'homme.

De par la composition de sa fraction insaponifiable riche en stérols, l'huile d'avocat raffinée de la présente invention peut participer favorablement au contrôle du taux de cholestérol sanguin. Ces propriétés rendent ainsi l'huile d'avocat raffinée particulièrement favorable à une utilisation comme huile alimentaire ou comme complément alimentaire. L'huile d'avocat raffinée de la présente invention est aussi avantageusement utilisée pour traiter les surcharges pondérales ou pour prévenir la prise de poids.

A ce titre, les compositions de la présente invention peuvent contenir en association avec l'huile d'avocat raffinée des molécules hypolipémiantes, telles que des fibrates (fénofibrate : agoniste PPAR-alpha), des inhibiteurs de l'HMG-CoA réducate (lovastatine, simvastatine). Les plantes hypoglycémiantes pouvant être également utilisées dans le cadre de la présente invention en association avec l'huile d'avocat raffinée sont avantageusement choisies dans le groupe constitué par le fenugrec (*Trigonella graenum*)*,* l'acide corosolique (composé actif des feuilles de l'arbre *Lagestroemia speciosa*)*,* le *Gymnema syllvestre,* le jus de fruit de momordique (*Momormodica charantia*)*,* l'eucalyptus (*Eucalyptus globulus*)*,* le *Panax ginseng,* ou les feuilles de myrtille (*Vaccinum myrtillus*)*.*

L'huile d'avocat raffinée ou les compositions selon la présente invention peut (peuvent) également être utilisée(s) à titre de médicament pour diverses applications thérapeutiques. L'huile d'avocat raffinée joue avantageusement un rôle essentiel dans la prévention ou le traitement des maladies cardiovasculaires, ou encore dans le contrôle du taux de cholestérol sanguin. L'huile d'avocat raffinée selon l'invention peut également être utilisée pour traiter l'obésité ou le diabète.

Dans le cadre de la prévention des maladies cardio-vasculaires, l'huile raffinée d'avocat peut-être utilisée en association avec des traitements anti-diabétique comme par exemple les traitements oraux du diabète. On peut citer l'insulinothérapie du diabète de type 2 et/ou des traitements divers tels que des capteurs de glucose et un pancréas artificiel. Comme exemple de traitement oral du diabète, on peut citer la stimulation de la sécrétion d'insuline (sulfamide hypoglycémiant ou apparenté (tolbutamide, carbutamide, glicazide, glimépiride, glipizide), dérivé de metformine, benfluorex), l'inhibition de l'alpha-glucosidase (acarbose et miglitol), le traitement de l'insulinorésistance (les thiazolidinediones (ou glitazone), tels que rosiglitazone et pioglitazone), ou les traitements de l'obésité comme les inhibiteurs de la recapture de la sérotonine (sibutramine), les inhibiteurs de la digestion des lipides (orlistat), les agonistes du récepteur Bêta3 adrénergique (augmentation de la lipolyse et de la thermogénèse), l'augmentation de l'utilisation périphérique du glucose par réduction de l'oxydation des acides gras), ou encore l'insulino-sécrétion avec le GP1, la pramlintide, l'IGF1 et les dérivés de vanadium, ou les glinides.

Dans le cadre de la prévention des maladies cardio-vasculaires, l'huile raffinée d'avocat peut-être utilisée en association avec des traitements anti-obésité. Les médicaments anti-obésité pouvant être utilisés, dans le cadre de la présente invention en association avec l'huile d'avocat raffinée sont avantageusement choisis dans le groupe constitué par l'orlistat (Xenical ®) et la sibutramine (Reductyl® ou Sibutral®).

Les nutriments anti-graisse pouvant être utilisés dans le cadre de la présente invention en association avec l'huile d'avocat raffinée, avantageusement avec un effet de synergie, sont avantageusement choisis dans le groupe constitué par des nutriments bloqueurs de l'absorption des graisses, tels que le chitosan, qui est une fibre extraite de l'exosquelette des crustacés, des nutriments capables d'augmenter la thermogénèse (« brûleur de graisse ») tels que l'éphédrine (herbe chinoise Ma Huang), la caféine, la théine et le citrus aurantium, le CLA (Acide linoléique conjugué issu de carthame préférentiellement), les huiles de poisson riches en oméga 3, de palme de cactus capteurs de lipides, les extraits secs des nutriments capables de réguler l'appétit (« coupe-faim ») tels que la L-phénylalanine et la L-tyrosine, des nutriments capables de réguler la glycémie, tels que des minéraux, par exemple le chrome ou le vanadium ou le magnésium, ou l'herbe ayurvédique Gymnema sylvestre, des inhibiteurs de la lipogénèse, tels que l'acide hydroxycitrique extrait du Garcinia cambodgia et des nutriments capables de transporter des graisses tels que la L-carnitine.

L'huile d'avocat raffinée ou les compositions selon la présente invention peut (peuvent) également être utilisée(s) à titre de médicament destiné à la réparation tissulaire, ou au traitement d'inflammations ou d'irritations de la peau, des phanères ou des muqueuses, en particulier pour traiter l'eczéma, le psoriasis, le prurit l'ichtyose, l'acné, la xérose, la dermatite atopique, les peaux atopiques ou encore les peaux allergiques.

L'huile d'avocat raffinée ou les compositions selon la présente invention peut (peuvent) également être utilisée(s) à titre de médicament dans des applications rhumatologiques ou dentaires, éventuellement en association avec un traitement via les substituts osseux.

On entend par substituts osseux par exemple des céramiques de phosphate de calcium, des ciments ioniques injectables, des matériaux composites, des substituts osseux d'origine animale, ou du corail. Ces composés sont avantageusement utilisés dans les applications suivantes: reconstruction des pertes de substance osseuse après reprise de prothèse totale de hanche, augmentation de la crête alvéolaire, comblements parodontaux, foraminotomie cervicale compressive, arthrodèse cervicale et vertébrale postérieure, comblement de la mastoïde, réparation de la base antérieure du crâne, comblement de zones de prises de greffes osseuse iliaques, correction des contours osseux faciaux, comblement des défauts osseux traumatiques et des fractures des os longs du membre inférieur.

En particulier, l'huile d'avocat raffinée ou les compositions selon la présente invention peut (peuvent) également être utilisée(s) pour traiter et/ou prévenir les pathologies articulaires, telles que l'arthrose, ou encore les maladies parodontales, telles que la gingivite ou la parodontite.

Dans le cadre des applications dentaires, l'huile d'avocat raffinée peut-être associée à des antibiotiques, des agents anti-plaques, des inhibiteurs de MMPs, ou des anti-inflammatoires.

L'huile d'avocat raffinée selon l'invention peut être avantageusement utilisée en association avec des extraits peptidiques d'avocat et/ou des sucres d'avocat, ou encore avec des anti-bactériens cutanés et buccaux, tels que les cathélicidines et les défensines, en particulier la cathélicidine humaine, les α-défensines, les β-défensines, notamment hBD-1, hBD-2, hBD-3, et hBD-4, ou encore d'autres peptides ou protéines, tels que l'adrénomedulline, la cystatine, ou l'inhibiteur spécifique de l'élastase, particulièrement particulièrement l'élafin (SKALP).

L'extrait peptidique d'avocat pouvant être utilisé en association avec l'huile d'avocat raffinée selon l'invention contient typiquement 2 à 10% en poids d'azote alpha-aminé, par rapport au poids de la matière sèche de l'extrait peptidique.

L'extrait peptidique d'avocat est susceptible d'être obtenu par un procédé comprenant les étapes suivantes :
- obtention d'un tourteau d'avocat, avantageusement du fruit de l'avocat, par séchage puis extraction de l'huile ; ensuite
- cryobroyage et délipidation complète dudit tourteau puis décantation, centrifugation et récupéreration du gâteau ; puis
- première hydrolyse en présence de glucanases, suivie d'une centrifugation et de l'élimination de la fraction soluble ;
- seconde hydrolyse en présence d'une ou plusieurs protéases, suivie d'une centrifugation et de l'élimination du culot ; ensuite
- concentration de la phase peptidique par nanofiltration et le cas échéant, décoloration en présence de charbon actif ; suivie
- d'une filtration simple (10 µm) puis d'une ultrafiltration (seuil de coupure de 10 kD) ; enfin
- le cas échéant, ajout de conservateur, microfiltration stérilisante finale (0,2 µm) et conditionnement.

Avantageusement, l'extrait peptidique d'avocat présente la composition suivante en acides aminés, en pourcentage en poids par rapport au poids total d'acides aminés :

| | |
|---|---|
| Alanine | 6,4 - 7,8 |
| Arginine | 4,7 - 5,7 |
| Acide aspartique | 10,3 - 12,7 |
| Cystine-cystéine | 2,9 - 3,5 |
| Acide glutamique | 13,0 - 15,8 |
| Glycine | 5,3 - 6,5 |
| Histidine | 2,2 - 2,6 |
| Isoleucine | 4,8 - 5,8 |
| Leucine | 7,6 - 9,4 |
| Lysine | 3,0 - 3,8 |
| Méthionine | 1,2 - 1,6 |
| Phénylalanine | 4,7 - 5,7 |
| Proline | 4,1- 5,2 |
| Sérine | 5,5 - 6,7 |
| Thréonine | 4,6 - 5,6 |
| Tyrosine | 3,6 - 4,4 |
| Valine | 5,8 - 7,2 |

Typiquement, les compositions selon l'invention contiennent de l'huile d'avocat raffinée, ainsi que 0,1 à 20% en poids sec d'extrait peptidique d'avocat, par rapport au poids total de la composition.

L'extrait de sucres d'avocat pouvant être utilisé en association avec l'huile d'avocat raffinée selon l'invention est typiquement un extrait hydrosoluble de sucres d'avocat, et contient avantageusement du D-mannoheptulose et/ou du perséitol.

Selon une variante avantageuse de l'invention, la composition comprend de l'huile d'avocat raffinée selon l'invention en association avec du D-mannoheptulose et/ou du perséitol (sucres en C7) ou d'un de leurs dérivés chimiques. La composition présente avantageusement une teneur de 0,1 à 30 % en poids sec de sucre d'avocat, par rapport au poids total de la composition.

L'extrait hydrosoluble de sucres d'avocat est susceptible d'être obtenu par un procédé comprenant les étapes successives suivantes :
- obtention d'un tourteau d'avocat, avantageusement du fruit de l'avocat, par séchage de l'avocat puis extraction des lipides (huile) ; ensuite
- cryobroyage et délipidation complète dudit tourteau, puis décantation et centrifugation afin de récupérer une fraction soluble riche en sucres en C7 (élimination du gâteau) ;
- déminéralisation sur résine ionique de ladite fraction soluble, obtenue à l'étape précédente ; puis
- ultrafiltration à 10 000 daltons ;
- le cas échéant, concentration par évaporation sous vide, ajout de conservateur, stérilisation par micro filtration (0,2 µm) et conditionnement.

L'huile d'avocat raffinée ou les compositions selon la présente invention peut (peuvent) également être utilisée(s) dans des compositions cosmétiques destinées à traiter, réparer ou protéger les peaux ou les muqueuses sèches, ainsi que les cheveux secs ou les cheveux ternes. L'huile ou les compositions selon la présente invention peut (peuvent) également être utilisée(s) pour le traitement cosmétique de troubles liés au vieillissement de la peau, des muqueuses voisines et/ou des phanères, pour le traitement de troubles de la peau; des muqueuses voisines et/ou des phanères résultant d'une exposition à un rayonnement actinique, notamment un rayonnement U.V, pour le traitement des troubles de la peau, des muqueuses voisines et/ou des phanères résultant d'un stress exogène (pollution, etc) ou endogène, ou encore à titre d'agents assouplissants, régénérant ou fortifiants pour les cheveux.

Les compositions selon la présente invention peuvent par exemple contenir des excipients cosmétiquement ou pharmaceutiquement acceptables, ainsi que des additifs cosmétiques conventionnels, notamment des filtres organiques ou minéraux UVB/UVA, des antioxydants, des anti-radicalaires, des protecteurs de la cellule irradiée, des substances naturelles ou synthétiques capables de stimuler la synthèse des lipides cutanés, ou des actifs anti-âge connus de l'homme du métier (rétinoïdes, vitamines, etc.).

Les exemples suivants sont donnés à titre non limitatif et illustrent la présente invention. Tous les pourcentages indiqués sont des pourcentages en poids.

### Exemple 1 :

*Matière première :* elle est constitué par des avocats frais de la variété Fuerté et d'origine sud africaine. La teneur moyenne en huile des avocats frais est égale à 16 % en poids.

*Méchage des fruits:* 20 kg d'avocat frais entier sont coupés en lamelles de 2 à 50 mm à l'aide d'un trancheur à disque, et répartis dans des clayettes sur une épaisseur de 4 à 5 cm. Le séchage est réalisé dans une étuve thermo-régulée à une température de 70°C, pendant 48 heures. Les fruits séchés sont ensuite broyés sur un broyeur à cylindres cannelés. Le poids des fruits secs récupérés est de 5,56 kg, soit 27,8 % de la matière de départ.

*Extraction de l'huile :* Cette opération est réalisée sur une presse de laboratoire type Komet. L'huile extraite est filtrée sur buchner, puis conditionnée sous azote à l'abri de la lumière et de l'humidité. 1750 g d'huile d'avocat brute ont été extraits, soit 31,5 % des fruits secs engagés.

L'huile brute obtenue est séparée en deux parties pour procéder à deux essais comparatifs de raffinage.

### a) procédé incluant une étape de distillation moléculaire :

*Fractionnement*/*concentration :* L'étape de concentration est réalisée en utilisant un distillateur moléculaire à film raclé, de type Leybold KDL 4. La température est fixée à 230°C et le vide à 10⁻³ mmHg. Le rendement en distillat de cette opération est de 10,4 %, et la fraction lourde représente 89,6 % de l'huile brute mise en oeuvre.

*Raffinage de la fraction lourde :* Dans un réacteur conique de 500 ml, 200g de la fraction lourde de l'huile d'avocat brute sont chauffés à 76°C sous agitation, puis 0,05% d'acide phosphorique sont ajoutés. Le contact est alors maintenu pendant 45 min sous agitation. Une solution de soude est ajoutée, et l'agitation est maintenue jusqu'à formation complète de savons. La phase inférieure contenant les savons est ensuite soutirée après décantation. La phase organique supérieure est lavée à l'eau jusqu'à pH neutre. Après séchage sous vide à 90°C, 3 % de charbon Norit SA4 et 5 % de terres décolorantes sont ajoutés et maintenus en contact sous agitation pendant 1/2h. Le mélange est ensuite filtré. L'huile d'avocat décolorée est refroidie progressivement sous agitation lente jusqu'à une température de 12 °C. L'huile est alors maintenue à cette température pendant 48 h, puis filtrée sous pression. L'huile d'avocat décolorée et frigélisée est ensuite soumise à une désodorisation à 180°C sous 4 mbar. La vapeur est injectée dans l'huile avec un débit de 0,4 1/min pendant 4 heures. Après séchage et refroidissement, l'huile d'avocat raffinée est obtenue.

*Conditionnement:* L'huile d'avocat raffinée (152 g) est alors conditionnée sous gaz inerte à l'abri de la lumière et de l'humidité.

### Analyse :

Densité : 0,914
Indice de réfraction : 1,471
Indice d'acide : 0,1 mg de KOH/g
Indice de peroxyde : 0,2 méq/kg
Tenue au froid : limpide après 2 h à -5°C
Couleur Gardner : 2,9
Teneur en insaponifiable 0,6 %

### Composition en acide gras:

- Acide palmitique 16 %
- Acide pamitoléique 7,2 %
- Acide oléique 60 %
- Acide linoléique 15,2 %
- Acide linolénique 0,5 %
- Acide arachidique 0,3 %

Persin : non détecté
Lipides furaniques de l'avocat : non détectés

### Composition de la fraction insaponifiable :

Teneur en stérols : 52,9 g/100g de fraction insaponifiable
Teneur en squalène 0,32 g/100g de fraction insaponifiable

### Composition relative en stérols :

Campéstérol : 4,6 %
Stigmastérol : 1,67 %
Bêta Sitostérol : 48,96 %

### b) Procédé n'incluant pas une étape de distillation moléculaire :

*Raffinage de l'huile d'avocat brute:* Dans un réacteur conique de 500 ml, 200g d'huile d'avocat brute sont chauffés à 76°C sous agitation, puis 0,05% d'acide phosphorique sont ajoutés. Le contact est alors maintenu pendant 45 min sous agitation. Une solution de soude est ajoutée, et l'agitation est maintenue jusqu'à formation complète de savons. La phase inférieure contenant les savons est ensuite soutirée après décantation. La phase organique supérieure est lavée à l'eau jusqu'à pH neutre. Après séchage sous vide à 90°C, 3 % de charbon Norit SA4 et 5 % de terres décolorantes sont ajoutés et maintenus en contact sous agitation pendant 1/2h. Le mélange est ensuite filtré. L'huile d'avocat décolorée est ensuite refroidie progressivement sous agitation lente jusqu'à une température de 12 °C. L'huile est alors maintenue à cette température pendant 48 h, puis filtrée sous pression.

L'huile d'avocat décolorée et frigélisée est soumise à une désodorisation à 180°C sous 4 mbar. La vapeur est injectée dans l'huile avec un débit de 0,4 l/min pendant 4 heures. Après séchage et refroidissement, l'huile d'avocat raffinée est obtenue.

*Conditionnement* : L'huile d'avocat raffinée (170 g) est alors conditionnée sous gaz inerte à l'abri de la lumière et de l'humidité.

### Analyse :

Couleur Gardner: 9.0
Teneur en insaponifiable 4,42 %

### Composition en acide gras :

- Acide palmitique 20,9 %
- Acide pamitoléique 8,8 %
- Acide oléique 58,4 %
- Acide linoléique 10,4 %
- Acide linolénique 0,6 %
- Acide arachidique 0,2 %

Acétogénines : 400 ppm
Lipides furaniques de l'avocat : 2,25 g/100g

### Composition de la fraction insaponifiable :

Teneur en stérols : 14,1 g/100g de fraction insaponifiable
Teneur en squalène 3,11 g/100g de fraction insaponifiable

### Composition relative en stérols :

Campéstérol : 8,19 %
Stigmastérol : 14.94 %
Bêta Sitostérol : 40,42 %

Il apparaît ainsi que le procédé selon la présente invention, comprenant notamment une étape de distillation moléculaire, permet d'aboutir à une huile exempte substantiellement d'acétogénines et de lipides furaniques, et facilite grandement la décoloration.

### Exemple 2 :

Analyses comparatives de la composition glycéridique des huiles obtenues par le procédé selon l'invention avec des huiles obtenues par un procédé de l'art antérieur incluant une extraction par pression à froid et un raffinage chimique :
Les essais 1 et 2 correspondent à deux huiles selon la présente invention, en mettant en oeuvre le procédé tel que décrit dans l'exemple 1, en partant de deux matières premières différentes.
Les échantillons 1 et 2 sont des échantillons d'huiles de l'art antérieur, qui peuvent être achetées dans le commerce.

Les analyses comparatives de composition glycéridique sont présentées dans le tableau 1 suivant :

**Tableau 1 :**

| Produit | Triglycérides | Diglycérides | Monoglycérides |
|---|---|---|---|
| Essai 1 | 97,43 % | 2,57 % | ND |
| Essai 2 | 97,81 % | 2,19 % | ND |
| Echantillon 1 | 89,04 % | 3,51 % | 6,39 % |
| Echantillon 2 | 89,04 % | 3,10 % | 5,20 % |

Ces analyses comparatives mettent en évidence l'impact de la distillation moléculaire utilisée pour produire les huiles des essais 1 et 2 (comme décrit dans l'exemple 1) sur le fractionnement des glycérides de l'huile d'avocat. Ce procédé met en évidence l'élimination des glycérides partiels (mono et di glycérides) et l'enrichissement en triglycérides.

## Revendications

1. Procédé d'obtention d'une huile d'avocat raffinée riche en triglycérides, **caractérisé en ce qu'**il comprend les étapes successives suivantes :
(1) la déshydratation contrôlée des avocats frais ou ayant subi des transformations préalables, avantageusement réalisée à une température comprise entre -50°C et 120°C,
(2) l'extraction de l'huile des fruits déshydratés,
(3) le fractionnement de l'huile en sa fraction riche en triglycérides, le fractionnement étant une cristallisation à froid, une distillation sous vide, ou une distillation moléculaire, puis
(4) le raffinage de la fraction de l'huile riche en triglycérides.

2. Procédé d'obtention d'une huile d'avocat raffinée riche en triglycérides selon la revendication 1, **caractérisé en ce que** la déshydratation (1) est choisie dans le groupe constitué par le séchage sous courant d'air chaud ou sous atmosphère contrôlée, le séchage à pression atmosphérique ou-sous vide, en couche épaisse ou couche mince, le séchage par micro-ondes, le séchage par pulvérisation, la lyophilisation et la déshydratation osmotique en solution ou en phase'solide.

3. Procédé d'obtention d'une huile d'avocat raffinée riche en triglycérides selon la revendication 2, **caractérisé en ce que** la déshydratation (1) consiste en un séchage en séchoirs ventilés, en couche mince et sous courant d'air chaud, avantageusement à une température comprise entre 70 et 80°C pendant 8 à 36 heures.

4. Procédé d'obtention d'une huile d'avocat raffinée riche en triglycérides selon l'une quelconque des revendications 1 à 3, **caractérisé en ce que** l'extraction (2) est effectuée par une pression à froid ou à l'aide d'un solvant à basse température.

5. Procédé d'obtention d'une huile d'avocat raffinée riche en triglycérides selon l'une quelconque des revendications précédentes, **caractérisé en ce que** le fractionnement (3) est une distillation moléculaire qui est réalisée à une température comprise entre 180 et 260°C en maintenant une pression comprise entre 0.133 et 1.33 Pa (10⁻³ et 10⁻² mmHg).

6. Procédé d'obtention d'une huile d'avocat raffinée riche en triglycérides selon l'une quelconque des revendications précédentes, **caractérisé en ce que** le fractionnement (3) est une distillation moléculaire qui est mise en oeuvre dans un dispositif choisi parmi les distillateurs moléculaires de type centrifuge et les distillateurs moléculaires du type à film raclé.

7. Procédé d'obtention d'une huile d'avocat raffinée riche en triglycérides selon l'une quelconque des revendications précédentes, **caractérisé en ce que** le raffinage (4) comprend au moins une des opérations suivantes : traitement acide, neutralisation chimique, décoloration, frigélisation et désodorisation.

8. Procédé d'obtention d'une huile d'avocat raffinée riche en triglycérides selon la revendication 7, **caractérisé en ce que** le raffinage (4) comprend au moins une frigélisation qui est réalisée à une température comprise entre 5 et 18 °C, avantageusement pendant 1 à 15 jours.

9. Procédé d'obtention d'une huile d'avocat raffinée riche en triglycérides selon la revendication 7 ou 8, **caractérisé en ce que** le raffinage (4) comprend au moins une désodorisation qui est réalisée à une température comprise entre 150 et 210 °C, avantageusement sous vide, sous courant de vapeur saturée ou d'azote.

10. Huile d'avocat raffinée riche en triglycérides susceptible d'être obtenue par le procédé selon l'une quelconque des revendications 1 à 9, **caractérisée en ce qu'**elle contient une fraction insaponifiable riche en stérols, et **en ce qu'**elle contient au plus 100 ppm d'acétogines et au plus 500 ppm de lipides furaniques, par rapport au poids total de l'huile.

11. Huile d'avocat raffinée riche en triglycérides selon la revendication 10, **caractérisée en ce que** sa fraction insaponifiable contient au moins 40 % en poids de stérols, avantageusement entre 45 et 70 % en poids de stérols, par rapport au poids total des insaponifiables.

12. Composition contenant une huile d'avocat raffinée riche en triglycérides selon l'une quelconque des revendications 10 à 11, avantageusement à une concentration comprise entre 5 et 95 % en poids, par rapport au poids total de la composition.

13. Composition selon la revendication 12, **caractérisée en ce qu'**elle comprend en outre au moins un composé choisi parmi les huiles végétales, les insaponifiables d'huile végétale, et leurs mélanges.

14. Composition selon la revendication 12 ou 13, **caractérisée en ce qu'**elle comprend en outre au moins un composé choisi parmi les sucres aminés tels que la glucosamine, les glycosaminoglycanes (GAG) tels que la chondroïtine sulfate, les analogues de glycosaminoglycanes, la pentosane, la S-adénosylméthionine (SAMe), l'adénosine, la superoxyde dismutase (SOD), la L-ergothionine, les collagènes de type Il, les hydrolysats de collagène (gélatine), la diacérine, J'acide arachadonique, la tétracycline, les composés analogues de la tétracycline, la doxycycline, l'hydroxyproline, et leurs mélanges.

15. Huile d'avocat raffinées selon l'une quelconque des revendications 10 à 11 ou composition selon l'une quelconque des revendications 12 à 14 pour son utilisation comme médicament, comme agent dermatologique, comme agent cosmétique, ou comme nutraceutique, à visée humaine ou animale, avantageusement dans la prévention ou le traitement des maladies cardiovasculaires, des pathologies articulaires telles que l'arthrose, des rhumatismes, des maladies parodontales, telles que la gingivite ou la parodontite, ou encore des inflammations.

## Claims

1. Process to obtain refined avocado oil rich in triglycerides, **characterized in that** it comprises the following successive steps:
(1) controlled dehydration of fresh or previously processed avocados, advantageously conducted at a temperature of between -50°C and 120°C,
(2) extracting the oil from the dehydrated fruits,
(3) fractioning the oil into its triglyceride-rich fraction, fractioning being cold crystallization, vacuum distillation or molecular distillation, then
(4) refining the triglyceride-rich oil fraction.

2. Process to obtain refined avocado oil rich in triglycerides according to claim 1, **characterized in that** dehydration (1) is chosen from the group consisting of drying under a stream of hot air or in a controlled atmosphere, drying at atmospheric pressure or vacuum drying, thick or thin layer drying, microwave drying, spray-drying, freeze-drying and osmotic dehydration in solution or solid phase.

3. Process to obtain refined avocado oil rich in triglycerides according to claim 2, **characterized in that** dehydration (1) consists of drying in ventilated dryers, in thin layer and under a stream of hot air, advantageously at a temperature of between 70 and 80°C for 8 to 36 hours.

4. Process to obtain refined avocado oil rich in triglycerides according to any of claims 1 to 3, **characterized in that** extraction (2) is conducted by cold pressing or using a solvent at low temperature.

5. Process to obtain refined avocado oil rich in triglycerides according to any of the preceding claims, **characterized in that** fractioning (3) is molecular distillation conducted at a temperature of between 180 and 260°C maintaining pressure at between 0.133 and 1.33 Pa (10⁻³ and 10⁻² mmHg).

6. Process to obtain refined avocado oil rich in triglycerides according to any of the preceding claims, **characterized in that** fractioning (3) is molecular distillation performed in a device chosen from among molecular distillers of centrifugal type and molecular distillers of scraped film type.

7. Process to obtain refined avocado oil rich in triglycerides according to any of the preceding claims, **characterized in that** refining (4) comprises at least one of the following operations: acid treatment, chemical neutralization, bleaching, winterization and deodorizing.

8. Process to obtain refined avocado oil rich in triglycerides according to claim 7, **characterized in that** refining (4) comprises at least one winterization conducted at a temperature of between 5 and 18°C advantageously for 1 to 15 days.

9. Process to obtain refined avocado oil rich in triglycerides according to claim 7 or 8, **characterized in that** refining (4) comprises at least one deodorization conducted at a temperature of between 150 and 210°C advantageously in a vacuum, under a stream of saturated vapour or of nitrogen.

10. Refined avocado oil rich in triglycerides able to be obtained using the process according to any of claims 1 to 9, **characterized in that** it contains an unsaponifiable fraction rich in sterols, and **in that** it contains no more than 100 ppm acetogenins and no more than 500 ppm furan lipids relative to the total weight of the oil.

11. Refined avocado oil rich in triglycerides according to claim 10, **characterized in that** its unsaponifiable fraction contains at least 40% sterols by weight, advantageously between 45 and 70% sterols by weight relative to the total weight of the unsaponifiables.

12. Composition containing a refined avocado oil rich in triglycerides according to either of claims 10 to 11, advantageously at a concentration of between 5 and 95 weight % relative to the total weight of the composition.

13. Composition according to claim 12, **characterized in that** it further comprises at least one compound chosen from among vegetable oils, vegetable oil unsaponifiables and their mixtures.

14. Composition according to claim 12 or 13, **characterized in that** it further comprises at least one compound chosen from among amino sugars such as glucosamine, glycosaminoglycans (GAG) such as chondroitin sulfate, the analogs of glycosaminoglycans, pentosan, S-adenosylmethionine (SAMe), adenosine, superoxide dismutase (SOD), L-ergothionine, type-II collagens, collagen hydrolysates (gelatin), diacerein, arachidonic acid, tetracycline, analog compounds of tetracycline, doxycycline, hydroxyproline and their mixtures.

15. Refined avocado oil according to either of claims 10 to 11 or composition according to any of claims 12 to 14 for its use as medicinal product, as dermatological agent, as cosmetic agent or as neutraceutical, in man or animal, advantageously for the prevention or treatment of cardiovascular disease, joint pathologies such as arthritis, rheumatism, periodontal diseases such as gingivitis or periodontitis, or inflammation.

## Patentansprüche

1. Verfahren zum Erhalt eines raffinierten, triglyceridreichen Avocadoöls, **dadurch gekennzeichnet, dass** es die folgenden aufeinander folgenden Schritte umfasst:
(1) kontrolliertes Entwässern der frischen oder vorherigen Umwandlungen unterzogenen Avocados, vorteilhafterweise durchgeführt bei einer Temperatur zwischen -50°C und 120°C,
(2) Extrahieren des Öls aus den entwässerten Früchten,
(3) Fraktionieren des Öls in seine triglyceridreiche Fraktion, wobei das Fraktionieren eine Kaltkristallisierung, eine Vakuumdestillierung oder eine Molekulardestillierung ist, dann
(4) Raffinieren der Fraktion des triglyceridreichen Öls.

2. Verfahren zum Erhalt eines raffinierten, triglyceridreichen Avocadoöls nach Anspruch 1, **dadurch gekennzeichnet, dass** die Entwässerung (1) aus der Gruppe ausgewählt wird, die aus der Trocknung unter einem heißen Luftstrom oder unter eine kontrollierten Atmosphäre, der Trocknung bei atmosphärischem Druck oder unter Vakuum, in dicker Schicht oder dünner Schicht, der Trocknung durch Mikrowellen, der Trocknung durch Zerstäubung, der Gefriertrocknung und der osmotischen Entwässerung in Lösung oder in der festen Phase besteht.

3. Verfahren zum Erhalt eines raffinierten, triglyceridreichen Avocadoöls nach Anspruch 2, **dadurch gekennzeichnet, dass** die Entwässerung (1) eine Trocknung in belüfteten Trocknern in dünner Schicht und unter heißem Luftstrom, vorteilhafterweise bei einer Temperatur zwischen 70 und 80°C während 8 bis 36 Stunden umfasst.

4. Verfahren zum Erhalt eines raffinierten, triglyceridreichen Avocadoöls nach einem der Ansprüche 1 bis 3, **dadurch gekennzeichnet, dass** die Extraktion (2) durch eine Kaltpressung oder mithilfe eines Lösemittels bei niedriger Temperatur erfolgt.

5. Verfahren zum Erhalt eines raffinierten, triglyceridreichen Avocadoöls nach einem der vorhergehenden Ansprüche, **dadurch gekennzeichnet, dass** die Fraktionierung (3) eine Molekulardestillation ist, die bei einer Temperatur zwischen 180 und 260°C unter Beibehaltung eines Drucks zwischen 0,133 und 1,33 Pa (10⁻³ und 10⁻² mmHg) durchgeführt wird.

6. Verfahren zum Erhalt eines raffinierten, triglyceridreichen Avocadoöls nach einem der vorhergehenden Ansprüche, **dadurch gekennzeichnet, dass** die Fraktionierung (3) eine Molekulardestillation ist, die in einer Vorrichtung, gewählt aus den Molekulardestillatoren des Typs Zentrifuge und den Molekulardestillatoren des Typs mit Rakelfilm, durchgeführt wird.

7. Verfahren zum Erhalt eines raffinierten, triglyceridreichen Avocadoöls nach einem der vorhergehenden Ansprüche, **dadurch gekennzeichnet, dass** die Raffinierung (4) mindestens einen der folgenden Vorgänge umfasst: Säurebehandlung, chemische Neutralisierung, Entfärbung, Entstearinierung und Desodorierung.

8. Verfahren zum Erhalt eines raffinierten, triglyceridreichen Avocadoöls nach Anspruch 7, **dadurch gekennzeichnet, dass** die Raffinierung (4) mindestens eine Entstearinierung umfasst, die bei einer Temperatur zwischen 5 und 18°C, vorteilhafterweise während 1 bis 15 Tagen durchgeführt wird.

9. Verfahren zum Erhalt eines raffinierten, triglyceridreichen Avocadoöls nach Anspruch 7 oder 8, **dadurch gekennzeichnet, dass** die Raffinierung (4) mindestens eine Desodorierung umfasst, die bei einer Temperatur zwischen 150 und 210°C, vorteilhafterweise unter Vakuum, unter gesättigtem oder Stickstoff-Dampfstrom durchgeführt wird.

10. Raffiniertes, triglyceridreiches Avocadoöls, das durch das Verfahren nach einem der Ansprüche 1 bis 9 erhalten werden kann, **dadurch gekennzeichnet, dass** es eine nicht verseifbare, sterolreiche Fraktion enthält, und **dadurch**, dass es höchstens 100 ppm Acetogene und höchstens 500 ppm Furanlipide im Verhältnis zum Gesamtgewicht des Öls enthält.

11. Raffiniertes, triglyceridreiches Avocadoöls nach Anspruch 10, **dadurch gekennzeichnet, dass** seine nicht verseifbare Fraktion mindestens 40 Gew.-% Sterole, vorteilhafterweise zwischen 45 und 70 Gew.-% Sterole im Verhältnis zum Gesamtgewicht der nicht verseifbaren Komponenten enthält.

12. Zusammensetzung, umfassend ein triglyceridreiches Avocadoöl nach einem der Ansprüche 10 bis 11, vorteilhafterweise mit einer Konzentration zwischen 5 und 95 Gew.-% im Verhältnis zum Gesamtgewicht der Zusammensetzung.

13. Zusammensetzung nach Anspruch 12, **dadurch gekennzeichnet, dass** sie außerdem mindestens eine Verbindung, ausgewählt unter den pflanzlichen Ölen, den nicht verseifbaren Komponenten des pflanzlichen Öls und ihren Mischungen umfasst.

14. Zusammensetzung nach Anspruch 12 oder 13, **dadurch gekennzeichnet, dass** sie außerdem mindestens eine Verbindung, ausgewählt unter den Aminozuckern wie z.B. dem Glykosamin, den Glykosaminoglykanen (GAG) wie z.B. dem Chondroitinsulfat, den Analogen von Glykosaminoglykanen, dem Pentosan, dem S-Adenosylmethionin (SAMe), dem Adenosin, der Superoxid-Dismutase (SOD), dem L-Ergothionin, den Kollagenen des Typs II, den Kollagenhydrolysaten (Gelatine), dem Diacerein, der Arachadonsäure, dem Tetracyclin, den analogen Verbindungen des Tetracyclin, dem Hydroxyprolin und ihren Mischungen, umfasst.

15. Raffiniertes Avocadoöl nach einem der Ansprüche 10 bis 11 oder Zusammensetzung nach einem der Ansprüche 12 bis 14 für ihre Verwendung als Medikament, als dermatologisches Mittel, als kosmetisches Mittel oder als nutrazeutisches Mittel zur Anwendung bei Menschen oder Tieren, vorteilhafterweise zur Prävention oder Behandlung von kardiovaskulären Erkrankungen, Gelenkerkrankungen wie z.B. Arthrose, Rheumatismen, Parodontalerkrankungen wie z.B. Gingivitis oder Parodontitis oder auch Entzündungen.
